# EUROPEAN PATENT APPLICATION

(11) **EP 4 656 639 A1**
(43) Date of publication of application: **03.12.2025**
(21) Application number: 24382579.1
(22) Date of filing: 30.05.2024
(51) Int. Cl.: C07D 471/18

(54) **METHOD FOR THE PREPARATION OF IBOGAMINE AND PURIFICATION OF IBOGAMINE AND IBOGAMINE-TYPE COMPOUNDS**

(71) Applicant: Oeri, Hans, Victoria, BC V8P 2L5 (CA); Kykeon Analytics Ltd, 28006 Madrid (ES)
(72) Inventor: OERI, Hans, Victoria, V8P 2L5 (CA); MARTINS, Daniel, Madrid (ES); SACRAMENTO BERGER, Rafael, Madrid (ES); BREINLINGER, Steffen, Madrid (ES)
(74) Representative: Elion IP, S.L.

(57) **Abstract**

The present disclosure is directed to a method for the preparation of ibogamine, in particular from ibogaine; to a method for purifying ibogamine and ibogamine-type compounds by Dry Column Vacuum Chromatography (DCVC); and to a method for crystallizing ibogamine, in particular free base ibogamine.

## Description

### TECHNICAL FIELD

The present disclosure relates to the field of organic synthesis and in particular is directed to a method for the preparation of ibogamine from ibogaine; to a method for purifying ibogamine by Dry Column Vacuum Chromatography (DCVC); and to a method for crystallizing ibogamine, in particular free base ibogamine.

### BACKGROUND

The exploration of novel compounds for addiction therapy and trauma therapy is a critical frontier in medical research, aiming to address the escalating global challenge of substance abuse and mental trauma. Among the promising candidates is ibogaine (12-methoxyibogamine), a psychoactive alkaloid derived from the Tabernanthe iboga plant, which has shown good results in the treatment of addiction. Ibogaine's unique pharmacological properties, including its ability to induce introspective and transformative experiences, have led to its application in therapy for addiction and trauma, particularly in the context of substance dependence.

While ibogaine has demonstrated encouraging results in addiction treatment, the need for enhanced derivatives with improved pharmacokinetic profiles, reduced side effects, and heightened therapeutic efficacy remains paramount.

Ibogamine is a derivative of ibogaine that has the potential to provide a more targeted and potent solution for addiction treatment with reduced toxic effects.

However, there are challenges in the preparation of ibogamine, in particular in obtaining a sufficiently pure ibogamine product suitable for pharmaceutical applications, in good yields which at the same time its preparation is scalable in order to be implemented at an industrial scale.

### SUMMARY

The present disclosure relates to a method for the preparation of ibogamine, which provides a streamlined three-step process to convert ibogaine into ibogamine. It has been found that with a method as described herein ibogamine can be achieved with a good overall yield and purity, highlighting the efficiency of the route developed which represents a significant milestone in the field of synthesis of ibogaine derivatives given its scalability and practical application in large-scale production.

In particular, the present disclosure relates to a method for the preparation of ibogamine comprising the steps of:
i) demethylating ibogaine by reacting ibogaine, with a dealkylating agent to provide nor-ibogaine;
ii) modifying nor-ibogaine by reacting nor-ibogaine provided in step i) with a triflating agent to provide 12-TfO-ibogamine; and
iii) eliminating the O-triflate group from 12-TfO-ibogamine by reacting 12-TfO-ibogamine provided in step ii) with a reducing agent to provide ibogamine, wherein the reducing agent comprises:
   - a palladium catalyst, and
   - a phosphine ligand.

The present disclosure also relates to a method for crystallizing free base ibogamine comprising:
- dissolving free base ibogamine in a solvent to provide a solution of ibogamine in the solvent;
   and
- crystallizing free base ibogamine from the solution of ibogamine in the solvent, to provide a crystallized free base ibogamine.

The present disclosure further relates to a method for purifying ibogamine and/or ibogamine-type compounds, by DCVC, comprising eluting ibogamine and/or ibogamine-type compounds in the presence of ammonia.

### DETAILED DESCRIPTION

As indicated above present disclosure relates to a method for the preparation of ibogamine comprising the steps of:
i) demethylating ibogaine by reacting ibogaine, with a dealkylating agent to provide nor-ibogaine;
ii) modifying nor-ibogaine by reacting nor-ibogaine provided in step i) with a triflating agent to provide 12-TfO-ibogamine; and
iii) eliminating the O-triflate group from 12-TfO-ibogamine by reacting 12-TfO-ibogamine provided in step ii) with a reducing agent to provide ibogamine, wherein the reducing agent comprises:
   - a palladium catalyst, and
   - a phosphine ligand.

The chemical structure, chemical names, IUPAC names and CAS Numbers of ibogaine, nor-ibogaine,12-TfO-ibogamine and ibogamine are detailed in the following table:

| **Chemical name; synonyms (if any); CAS number (if any); IUPAC name** | **Chemical structure** |
|---|---|
| Ibogaine; 12-methoxyibogamine; 83-74-9; (1R,15R,17S,18S)-17-ethyl-7-methoxy-3,13-diazapentacyclo-[13.3.1.0^{2,10}.0^{4,9}.0^{13,18}]nonadeca -2(10),4(9),5,7-tetraene | 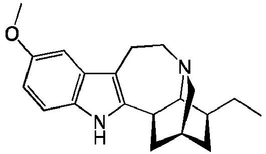 |
| Nor-ibogaine; 12-hydroxyibogamine; 481-88-9; (1R,15S,17S,18S)-17-ethyl-3,13-diazapentacyclo-[13.3.1.0^{2,10}.0^{4,9}.0^{13,18}]nonadeca-2(10),4(9),5,7-tetraen-7-ol | 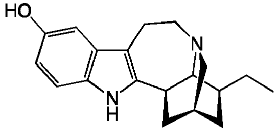 |
| 12-TfO-ibogamine; 12-O-[(trifluoromethyl)sulfonyl]ibogamine; - ; (1R,15S,17S,18S)-17-ethyl-3,13-diazapentacyclo [13.3.1.0^{2,10}.0^{4,9}.0^{13,18}] nonadeca-2(10),4(9),5,7-tetraene-7-trifluoromethanesulfonate | 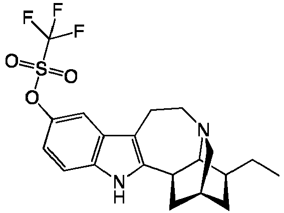 |
| Ibogamine; - ; 481-87-8; (1R,15R,17S,18S)-17-ethyl-3,13-diazapentacyclo [13.3.1.0^{2,10}.0^{4,9}.0^{13,18}]nonadeca-2(10),4,6,8-tetraene | 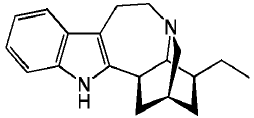 |

Ibogamine-type compounds, including ibogamine, ibogaine and ibogaine derivatives, are presented in the above table in their free base form, i.e., with their basic nitrogen not being protonated and, therefore, as the neutral compounds. Salts of ibogamine-type compounds such as chloride salts of the protonated compounds are typically represented alongside hydrochloride (HCl), without specifying the position of the proton in the molecule (typically protonated on the nitrogen of the tertiary amine of the fused ring) and are referred to as, e.g., ibogaine hydrochloride (salt), ibogamine hydrochloride (salt), etc...

Step i) of a method for the preparation of ibogamine as described herein comprises demethylating ibogaine. Ibogaine is commercially available and may also be prepared by methods known in the art. In a method described herein ibogaine may be used in any suitable form. For instance, ibogaine may be used in its free base form or in the form of its hydrochloride salt (ibogaine hydrochloride). Ibogaine hydrochloride may be preferably used as a starting material.

Demethylating ibogaine to provide nor-ibogaine is achieved by reacting ibogaine with a dealkylating agent. For instance, ibogaine may be mixed with the dealkylating agent to form a reaction mixture.

Ibogaine and nor-ibogaine have the chemical structure and alternative chemical names indicated in the table above.

A dealkylating agent is understood herein as any chemical compound or compound mixture that is suitable for removing an alkyl group, in particular from an O-alkyl group for liberating an OH group.

A suitable dealkylating agent may be selected from boron tribromide (BBr₃), aluminium iodide (AlI₃), aluminum bromide (AlBr₃), aluminum chloride (AlCl₃), pyridinium chloride (py+HCl), pyridinium bromide (py+HBr), iodotrimethylsilane (TMSI), and lithium diphenylphosphide (C₁₂H₁₀)PLi. Preferably the dealkylating agent may be BBr₃. Advantageously, BBr₃ has been found to provide the product in good yields and at room temperature. BBr₃ may be preferred due to its ability to perform demethylation under mild conditions (at or below room temperature) without the need for strong acids or bases. Additionally, it has been found to offer high selectivity and to tolerate a variety of other functional groups.

Demethylating may be performed by reacting ibogaine with the dealkylating agent in the presence of a solvent. For instance, a suitable solvent may be selected from dichloromethane, chloroform, acetonitrile, toluene, and hexane. Preferably the solvent may be dichloromethane. Advantagously, dichloromethane has been found to be able to solvate protonated ibogaine and noribogaine, has a low boiling point that facilitates removal by evaporation, has a low cost and is easy to handle.

Reacting ibogaine with the dealkylating agent, may be performed under anhydrous conditions, e.g., using an anhydrous solvent such as anhydrous dichloromethane. This may be achieved, e.g., by placing all glassware that is part of the apparatus used in a drying oven set to approximately 120°C for at least 24 hours before use, and by using an inert atmosphere, such as using a nitrogen or an argon atmosphere. Anhydrous conditions may be required if working with a dealkylating agent sensitive to water, such as BBr₃ as this reagent decomposes upon contact with water.

In several embodiments, ibogaine may be mixed with a solvent to provide a mixture of ibogaine in the solvent in the form of a suspension or a solution of ibogaine in the solvent, preferably in the form of a suspension. The dealkylating agent may be added to the mixture of ibogaine in the solvent, providing the reaction mixture.

The dealkylating agent may be used in a molar ratio amount of ibogaine to dealkylating agent of above 1.0:1.0, in particular of at least 1.0:1.3, more in particular at least 1.0:1.5. For instance, suitable molar ratios of ibogaine to dealkylating agent may be selected from 1.0:1.0 to 1.0:3.0, in particular from 1.0:1.3 to 1.0:2.0, and more in particular from 1:1.5 to 1:1.8. It has been found that molar ratios within these ranges contribute to providing a nor-ibogaine product of higher purity than, e.g., if lower molar ratios are used.

Demethylation may be performed, e.g., under mixing at any suitable temperature, e.g., at room temperature such as a temperature from 18 to 28 °C, in particular from 20 to 25 °C.

Demethylation may be monitored by means known in the art, e.g., by chromatography such as thin layer chromatography, and the reaction may be allowed to proceed until, e.g., the starting material is no longer observed. Typical reaction times may vary from, e.g., 10 to 96 hours, in particular from 24 to 72 hours, and more in particular from 48 to 65 hours.

Once the reaction is deemed complete, the product of the reaction (nor-ibogaine) may be isolated from the reaction mixture.

Isolating nor-ibogaine from the reaction mixture may comprise quenching the reaction mixture with addition of a solvent, such as methanol, to the reaction mixture, thereby providing a quenched reaction mixture.

Isolation may comprise one or more washing and/or extracting steps using a suitable solvent system including any solvent already present in the reaction mixture and any solvent added to the reaction mixture. A suitable solvent system may comprise an aqueous solvent and/or an organic solvent, typically both an aqueous solvent and an organic solvent. An aqueous solvent may be an aqueous solution such as a basic and/or an acid aqueous solution, e.g., an aqueous solution selected from an aqueous sodium hydrogen carbonate solution (e.g., 0.1 M solution of sodium hydrogen carbonate) and/or an aqueous hydrochloric acid solution (e.g., 1 M hydrochloric acid solution). An organic solvent may be selected from, e.g., ethyl acetate, diethyl ether and any organic solvent already present in the reaction mixture, e.g., dichloromethane, chloroform, acetonitrile, toluene, and hexane as detailed above.

Extracting and/or washing using aqueous and/or organic solvents may typically result in one or more aqueous phase and/or organic phase. If desired, the pH of the aqueous phase may be adjusted during isolation, e.g., by using a basic aqueous solution or an acidic aqueous solution or changing the pH of the aqueous phase by adding an appropriate amount of base (e.g., NaHCO₃) or acid (e.g., HCl). Whether nor-ibogaine is obtained in the form of a free base or a salt may depend on the pH of the aqueous phase during isolation.

In several particular embodiments, in step i) the reaction mixture may be quenched with a solvent such as methanol, e.g., when the reaction is deemed complete. The pH of quenched reaction mixture may be adjusted, e.g., to a basic pH, in particular, to a pH of about 10 by addition of, e.g., a basic aqueous solution (such as a 0.1 M sodium hydrogen carbonate aqueous solution), forming an aqueous phase and organic phase and, optionally, a solid phase. The organic phase, and any solid phase, may then be separated from the aqueous phase. The organic phase may be evaporated to provide a residue, the residue obtained may be dissolved in, e.g., an acid aqueous solution (such as a 1 M hydrochloric acid aqueous solution). The aqueous phase may be washed with organic solvent, e.g. dichloromethane, diethyl ether, and then the pH of aqueous phase may be adjusted, in particular, to pH of about 10. The aqueous phase may be extracted with an organic solvent such as ethyl acetate. Nor-ibogaine, preferably in the form of free base, may then be isolated by evaporation of the organic solvent.

Nor-ibogaine may be provided, e.g., isolated from the reaction mixture, in the form of a free base or of a salt, such as nor-ibogaine hydrochloride. If desired, hydrochloride salts of nor-ibogaine may be prepared by dissolving nor-ibogaine free base (e.g., isolated as an oil) in an alcohol, e.g. methanol, 1-propanol, neutralizing with, e.g., ethereal hydrochloric acid 2M and adding diethyl ether, petroleum ether or hexane. Thereby crystals of nor-ibogaine hydrochloride may be formed which may be collected by filtration and air-dried.Step ii) of a method for the preparation of ibogamine as described herein comprises modifying nor-ibogaine by reacting nor-ibogaine provided in step i) with a triflating agent to provide 12-TfO-ibogamine.

12-TfO-ibogamine refers to triflate ibogamine having a O-triflate group (TfO) on the 12^{th} position of ibogamine, and has the chemical structure and alternative chemical names indicated in the table above.

The triflating agent may be selected from, e.g., *N*-phenyl-bis(trifluoromethanesulfonimide), *N*-(2-pyridyl)triflimide, *N*-phenyltriflimide, imidazole triflate, *N*-(2-pyridyl) triflimide, and preferably the triflating agent is *N*-phenyl-bis(trifluoromethanesulfonimide).

In step ii) nor-ibogaine may be used in any suitable form, e.g., as a free base or as a salt, such as nor-ibogaine hydrochloride, preferably in the form of a free base.

Modifying nor-ibogaine may be performed by reacting nor-ibogaine with the triflating agent in the presence of a solvent. For instance, a suitable solvent may be selected from dichloromethane, chloroform, acetonitrile, toluene, and hexane. Preferably the solvent may be dichloromethane.

In several embodiments, nor-ibogaine may be mixed with a solvent to provide a mixture of nor-ibogaine in the solvent in the form of, e.g., a suspension or a solution of noribogaine in the solvent, preferably in the form of a solution. The triflating agent may be added to the mixture of nor-ibogaine in the solvent, providing a reaction mixture.

Modification of nor-ibogaine may be performed under mixing at any suitable temperature, e.g., at room temperature such as a temperature from 18 to 28 °C, in particular from 20 to 25 °C.

Modification of nor-ibogaine may be monitored by means known in the art, e.g., by chromatography such as thin layer chromatography, and the reaction may be allowed to proceed until, e.g., the starting material is no longer observed. Typical reaction times may vary from, e.g., 1 to 30 hours, in particular from 5 to 25 hours, and more in particular from 12 to 15 hours.

Once the reaction is deemed complete, the product of the reaction (12-TfO-ibogamine) may be isolated from the reaction mixture.

Isolation may comprise one or more washing and/or extracting steps using a suitable solvent system, including any solvents present in the reaction mixture. Isolation may also optionally comprise the purification of reaction product (12-TfO-ibogamine), e.g., to provide purified 12-TfO-ibogamine.

A suitable solvent system for washing and/or extracting may comprise an aqueous solvent and/or an organic solvent, typically both an aqueous solvent and an organic solvent. An aqueous solvent may be an aqueous solution such as a basic and/or an acid aqueous solution, e.g., an aqueous solution selected from an aqueous sodium hydrogen carbonate solution (e.g., a 10% w/w aqueous solution of sodium hydrogen carbonate, based on the total weight of the aqueous solution). An organic solvent may be selected from, e.g., dichloromethane, diethyl ether, ethyl acetate. Preferably the solvent may be dichloromethane.

In several particular embodiments, in step ii) the reaction mixture comprising an organic solvent, such as dichloromethane, may be washed by addition of, e.g., a basic aqueous solution (such as a 10% sodium hydrogen carbonate aqueous solution), e.g. once the reaction is deemed complete. Washing may provide an aqueous phase and organic phase and, optionally, a solid phase. The organic phase, and any solid phase, may then be separated from the aqueous phase. The product of the reaction may be isolated by evaporating the organic solvent to provide 12-TfO-ibogamine, preferably in the form of a free base. The product of the reaction (12-TfO-ibogamine), may be purified by DVCV, e.g., as described below.

Step iii) of a method as described herein comprises eliminating the O-triflate group from 12-TfO-ibogamine by reacting 12-TfO-ibogamine provided in step ii) with a reducing agent to provide ibogamine. The reducing agent comprises: a palladium catalyst and a phosphine ligand. In several embodiments, the molar ratio of the phosphine ligand to palladium catalyst may be from 0.5:1.0 to 1.0:1.5, more in particular of about 1.0:1.0.

A palladium catalyst may be preferably selected from palladium acetate [Pd(OAc)₂], palladium bis(dibenzylideneacetone) [Pd(dba)₂], tris(dibenzylideneacetone)dipalladium [Pd₂(dba)₃], dipalladium-tris(dibenzylideneacetone)chloroform complex [Pd₂(dba)₃ CHCl₃], and more preferably the palladium catalyst may be palladium acetate, due its chemical stability and good process efficiency.

A phosphine ligand, may be preferably selected from 1,1'-bis(diphenylphosphino)-ferrocene (DPPF), 1,2'-bis(diphenylphosphino)-ethane (DPPE), 1,3'-bis(diphenylphosphino)-propane (DPPP), 1,4'-bis(diphenylphosphino)-butane, and more preferably the phosphine ligand is 1,1'-bis(diphenylphosphino)-ferrocene due its good catalytic performance.

In step iii) the molar ratio of the phosphine ligand (e.g., a phosphine ligand as described above such as 1,1'-bis(diphenylphosphino)-ferrocene) to 12-TfO-ibogamine may be from 0.001:1.0 to 0.12:1.0, preferably from 0.01:1.0 to 0.10:1.0, more preferably from 0.01:1.0 to 0.09:1.0, and yet more preferably from 0.05:1.0 to 0.08:1.0. It has been surprisingly found that using a reducing agent in such ratios, advantageously results in a product with a higher purity and yield when compared to, e.g., higher ratios of reducing agent.

In step iii) 12-TfO-ibogamine may be used in any suitable form, e.g., as a free base or as a salt, such as 12-TfO-ibogamine hydrochloride, preferably in the form of a free base.

Eliminating the O-triflate group from 12-TfO-ibogamine may be performed by reacting 12-TfO-ibogamine with the reducing agent in the presence of a solvent. For instance, a suitable solvent may be selected from dimethylformamide (DMF), toluene, and benzene. Preferably the solvent may be dimethylformamide.

In several embodiments, 12-TfO-ibogamine may be mixed with a solvent to provide a mixture of 12-TfO-ibogamine in the solvent in the form of a suspension or a solution of 12-TfO-ibogamine in the solvent, preferably in the form of a solution.

The reducing agent may be added to the mixture of 12-TfO-ibogamine in the solvent, providing a reaction mixture.

Eliminating the O-triflate group from 12-TfO-ibogamine may be performed under mixing at any suitable temperature, e.g., at room temperature such as a temperature from 18 to 28 °C, in particular from 20 to 25 °C. In several embodiments the temperature may be raised to a temperature from 30 to 100 °C, in particular from 40 to 80 °C, and more in particular from 50 to 70 °C, e.g., about 60 °C.

Eliminating the O-triflate group from 12-TfO-ibogamine may be monitored by means known in the art, e.g., by chromatography such as thin layer chromatography, and the reaction may be allowed to proceed until, e.g., the starting material is no longer observed. Typical reaction times may vary from, e.g., 1 to 60 hours, in particular from 5 to 50 hours, and more in particular from 10 to 30 hours.

Once the reaction is deemed complete, the product of the reaction (ibogamine) may be isolated from the reaction mixture. Isolation may comprise evaporation of solvent, e.g., present in the reaction mixture. Isolation may optionally comprise purification of the product of the reaction (ibogamine), e.g., to provide purified ibogamine.

In several particular embodiments, in step iii) the product of the reaction (ibogamine) may be isolated from the reaction mixture comprising an organic solvent, such as DMF, by evaporating the organic solvent to provide ibogamine, preferably in the form of the free base. Ibogamine may then be purified by, e.g., DCVC as detailed below.

In several embodiments, step ii) and/or step iii) of a method for the preparation of ibogamine as described herein may comprise purifying 12-TfO-ibogamine and/or ibogamine by, e.g., DCVC, to provide purified 12-TfO-ibogamine and/or purified ibogamine.

DCVC is a chromatographic technique useful for the purification of certain organic compounds. For instance, Pedersen et al (Synthesis 2001 (16), 2431-2434; [DOI: 10.1055/s-2001-18722]) describe DCVC as an alternative to the commonly used Flash Column Chromatography for purification in organic synthesis that has an excellent resolving power, is easily applied to large scale chromatography (up to 100 g), is fast and provides significant reductions in solvent and the amount of silica used, e.g., when compared to other methods such as Flash Column Chromatography.

It has now been found that DCVC can be suitably and advantageously used for the purification of ibogamine-type compounds. Accordingly, the present disclosure is also directed to a method for purifying ibogamine and/or an ibogamine-type compound selected from ibogaine, nor-ibogaine or 12-TfO-ibogamine, preferably for purifying ibogamine and/or 12-TfO-ibogamine, by DCVC. ibogamine and ibogamine-type compounds may be preferably purified by DCVC in their free base form.

Purifying by DCVC may comprise eluting ibogamine and/or the ibogamine-type compounds in the presence of ammonia. Eluting may be performed with a solvent system comprising a solvent and ammonia. Preferably the solvent may comprise from 0.005 to 1 % v/v of ammonia, preferably from 0.05 to 0.5 % v/v and more preferably 0.08 to 0.2 % v/v of ammonia. The % v/v is based on the total volume of solvent. The solvent may be preferably selected from ethyl acetate and n-heptane. In several particular embodiments, eluting may use a gradient of ethyl acetate:n-heptane, e.g., from 10:90 to 100:0, in particular from 30:70 to 100:0. The increase of ethyl acetate may be performed in steps, e.g., from 5% to 10%.

In a method for preparing ibogamine, purifying 12-TfO-ibogamine and/or ibogamine by DCVC of steps ii) and/or iii) may also be performed as detailed above.

In several embodiments, a method for preparing ibogamine as described herein may comprise after step iii) a further step iv) of crystallizing ibogamine or purified ibogamine provided in step iii) to provide a crystallized ibogamine.

In a method as described herein the ibogamine or the purified ibogamine provided in step iii) and/or the crystallized ibogamine provided in step iv) is free base ibogamine.

Crystallizing of step iv) may comprise:
- dissolving the ibogamine or the purified ibogamine obtained in step iii) in a solvent to provide a solution of ibogamine in the solvent;
   and
- crystallizing ibogamine from the solution of ibogamine in the solvent, to provide the crystallized ibogamine.

The solvent to which ibogamine may be dissolved for crystallizing may preferably be an alcohol, more preferably an alcohol selected from methanol, ethanol, isopropyl alcohol, and yet more preferably the solvent may be methanol.

In step iv) ibogamine free base may be dissolved in the solvent at any suitable temperature to provide a solution of ibogamine in the solvent. A temperature from 30 to 80 °C may be preferred, more preferably from 40 to 60°C. Such temperatures may advantageously facilitate dissolution of ibogamine and any impurities without adversely affecting the stability of ibogamine.

In step iv) ibogamine may be crystallized from the solution of ibogamine in the solvent by, e.g., cooling the solution, preferably cooling the solution to a temperature from -50 to 0 °C, preferably from -40 to -5 °C, and more preferably from -30 to -10 °C.

If desired, crystallized ibogamine may be recrystallized one or more times, which may further improve its purity. Nonetheless, crystallization of ibogamine as described herein already results in a product of high purity and recrystallization may not be required.

Ibogamine may be preferably crystallized in the form of a free base. It has been surprisingly found that, unlike other organic iboga-type compounds, the crystallization of ibogamine is improved, leading to higher purity and yields, if ibogamine is crystallized in its free base form, instead of as a salt, such as an ibogamine hydrochloride salt.

Accordingly, the present disclosure also relates to a method for crystallizing free base ibogamine. The method for crystallizing free base ibogamine may be performed as described above for crystallizing ibogamine of step iv) of a method for the preparation of ibogamine as described above.

Methods as described herein have been found to provide ibogamine in high purity and high yields. In particular, the method for the preparation of ibogamnie comprising steps i), ii), iii) and optionally step iv) as described herein may provide ibogamine from ibogaine in overall yields from 30 to 80 %, based on the total moles of ibogaine used as starting materials and moles of ibogamine obtained at the end of the method, in particular a yield from 40 to 70 % and yet more in particular from 45 to 65 %. The method may provide ibogamine having a purity from 90 to 98 % as determined by, e.g., Liquid cromathography - mass spectrometry (LC-MS), in particular ibogamine may have a purity from 93 to 98 %, and more in particular from 95 to 97 %.

The methods of purification by DCVC and of crystallization have also been found to provide ibogamine or ibogamine-type compounds of high purity with high yields.

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc.

On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

The present disclosure is further illustrated by the following examples without being limited thereto or thereby.

### EXAMPLES

### General materials and methods

Ibogaine hydrochloride, *N*-phenyl-bis(trifluoromethanesulfonimide), 1,1'-bis(diphenylphosphino)-ferrocene, palladium acetate, boron tribromide (BBr3), sodium carbonate (Na₂CO₃), sodium hydrogen carbonate (NaHCO₃), anhydrous magnesium sulphate (MgSO₄), hydrochloric acid (HCl), formic acid, dichloromethane (DCM), methanol (MeOH), ethyl acetate (AcOEt), heptane, ammonia, triethylamine (Et₃N), dimethylformamide (DMF) were purchased from Merck KGaA, Darmstadt, Germany.

All reactions were monitored by thin-layer chromatography (TLC), performed on 0.2 mm Merck aluminium sheets precoated with silica gel 60 F254. TLC plates were visualized under a UVITEC LF 215LS (UVITEC, Cambridge, UK) light with a wavelength of 254 and/or 365 nm.

The solvents were evaporated in a rotary vacuum evaporator Büchi R205 Professional Rotary Evaporator (BÜCHI Labortechnik AG, Flawil, Switzerland) and the compounds were dried in an Eppendorf Concentrator 5301 (Eppendorf, Hamburg, Germany).

¹H and ¹³C nuclear magnetic resonance (NMR) data was recorded, on a Benchtop NMR 100Mhz PRO (Nanalysis, Calgary, Canada) operating at 100 Mega-Hertz (MHz). Assignments were also made from distortionless enhancement by polarization transfer (DEPT) experiments. In the ¹H-NMR spectra, the chemical shift (δ) values were obtained in ppm relative to tetramethylsilane (TMS), along with the multiplicity of the signal, the number of protons and coupling constants, expressed in Hertz (Hz). In ¹³C-NMR spectra, the chemical shift (δ) values were obtained in ppm.

Electron spray mass-spectra (ESI-MS) were carried out on an Agilent 1260 Infinity II (Agilent Technologies, Inc, Santa Clara, USA) high pressure liquid chromatography and Agilent Ultivo QQQ (Agilent Technologies, Inc, Santa Clara, USA) mass spectrometer. The MS data were obtained as m/z (% of the relative intensity of the most important fragments).

### Example 1: Preparation of nor-ibogaine from ibogaine

Nor-ibogaine (i.e., 12-hydroxyibogamine) was prepared from ibogaine by demethylation using boron tribromide (BBr₃) as dealkylating agent.

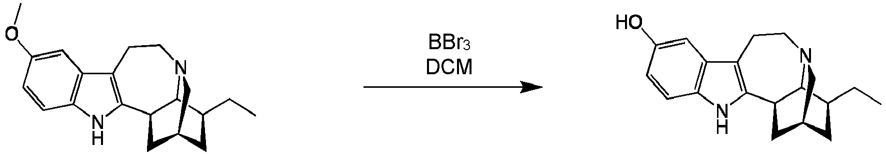

In a three-neck round-bottom 1000 mL flask, a solution of boron tribromide 0.9 M in anhydrous dichloromethane (31.5 mmol) was added dropwise, under argon, to a stirred suspension of ibogaine hydrochloride (19.9 mmol) in anhydrous dichloromethane (600.0 mL). The mixture was stirred, and reaction progression monitored by thin layer chromatography. After 65 hours, the reaction was quenched through addition of 35.0 mL of methanol. The remaining product was dissolved adding a solution of 0.1 M aqueous solution of sodium hydrogen carbonate, forming a cloudy solution of a precipitate that did not dissolve in either phase. The obtained mixture was washed (3 x 100.0 mL) with 0.1 M aqueous solution of sodium hydrogen carbonate. After separating the phases (the solid precipitate, the aqueous phase, and the organic phase) the solid precipitate was redissolved in 1 M hydrochloric acid aqueous solution and washed with ethyl acetate (3 x 100.0 mL); and the organic phase was evaporated, and the remaining residue was redissolved in 0.1 M aqueous solution of sodium hydrogen carbonate. All the aqueous phases obtained were combined and the pH adjusted to 10. The target compound (nor-ibogaine) was extracted from the pH adjusted-combined aqueous phases as a free base with ethyl acetate (3 x 100.0 mL). The organic layer was dried over anhydrous magnesium sulphate and filtered. Evaporation of ethyl acetate revealed 5.47 g (18.5 mmol of target compound in the form of free base nor-ibogaine, in a yield of η=93.0%. The prepared compound was structurally characterized by NMR, LC-MS and FTIR (data not shown).

To obtain the hydrochloride salt of nor-ibogaine, the free base oil was dissolved isopropanol, neutralized with ethereal hydrochloric acid 2M and diethyl ether was added until the formation of crystals. The formed crystals were filtered and air dried.

### Example 2: Preparation of 12-TfO-ibogamine from nor-ibogaine

12-TfO-ibogamine was prepared from nor-ibogaine by modifying nor-ibogaine with *N*-phenyl-bis(trifluoromethanesulfonimide) as a triflating agent.

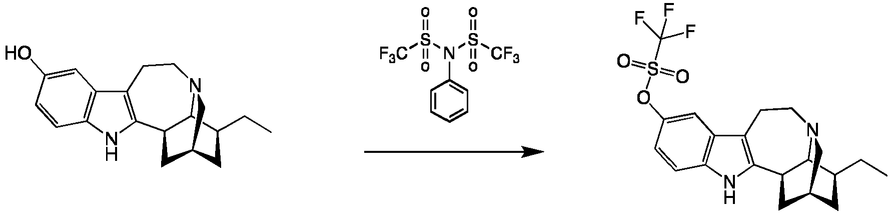

In a three-neck round-bottom 500 mL flask, triethylamine (107.6 mmol) was added to a solution of nor-ibogaine hydrochloride (16.3 mmol) in dichloromethane (350 mL). Subsequently, with agitation, *N*-phenyl-bis(trifluoromethanesulfonimide) (19.9 mmol) as triflating agent was carefully added. The mixture was stirred for 15 hours, and the completion of reaction monitored by thin layer chromatography. The obtained mixture was washed (3 x 75.0 mL) with 10% aqueous sodium hydrogen carbonate solution. The organic layer was dried over anhydrous magnesium sulphate, filtered over celite and evaporated. The product was purified by DCVC (silica, ethyl acetate/heptane/ammonia (from 10:90 to 100:0, in 0.1% ammonia) providing 6.37g (14.87 mmol, η=91.2%) of final product (free base 12-TfO-ibogamine). The prepared compound was structurally characterized by NMR, LC-MS and FTIR (data not shown).

### Example 3: Preparation of ibogamine from 12-TfO-ibogamine

Ibogamine was prepared from 12-TfO-ibogamine by eliminating the O-triflate group with a reducing agent comprising palladium acetate and 1,1'-bis(diphenylphosphino)-ferrocene.

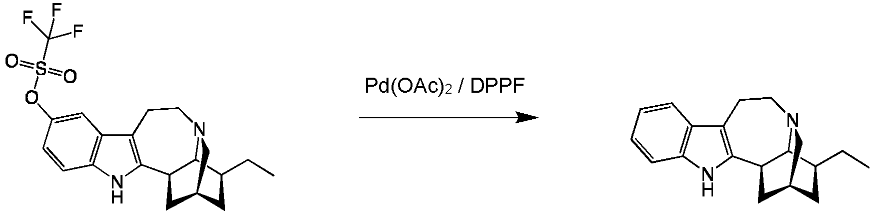

In a three-neck round-bottom 250 mL flask, triethylamine (78.6 mmol) and formic acid (51.8 mmol) was added to free base 12-TfO-ibogamine (20.3 mmol) dissolved in dimethylformamide (65 mL). Subsequently, 1,1'-bis(diphenylphosphino)-ferrocene (1.7 mmol) and palladium acetate (2.2 mmol) was added. The mixture was stirred for 15 hours, and the completion of reaction monitored by thin layer chromatography. The temperature was raised to 60 °C and the reaction was kept in agitation for an additional 12 hours. The consumption of starting material was monitored by thin layer chromatography (acetone/heptane/ammonia (30:70:0.1)). After completion, the dimethylformamide was evaporated. The product was purified by DCVC (silica, ethyl acetate/heptane (from 30:70 to 100:0, in 0.1% ammonia). Recrystallization from hot methanol (at a temperature of 60°C)) afforded 3.56 g (12.7 mmol, η=62.3%) of off-white crystals of target compound. The prepared compound was structurally characterized by NMR, LC-MS and FTIR (data not shown). The obtained product had a purity of 97% as determined by liquid cromathography-mass spectrometry.

## Claims

1. A method for the preparation of ibogamine comprising the steps of:
i) demethylating ibogaine by reacting ibogaine with a dealkylating agent to provide nor-ibogaine;
ii) modifying nor-ibogaine by reacting nor-ibogaine provided in step i) with a triflating agent to provide 12-TfO-ibogamine; and
iii) eliminating the O-triflate group from 12-TfO-ibogamine by reacting 12-TfO-ibogamine provided in step ii) with a reducing agent to provide ibogamine, wherein the reducing agent comprises:
- a palladium catalyst, preferably selected from palladium acetate [Pd(OAc)₂], palladium bis(dibenzylideneacetone) [Pd(dba)₂], tris(dibenzylideneacetone)dipalladium [Pd₂(dba)₃], dipalladium-tris(dibenzylideneacetone)chloroform complex [Pd₂(dba)₃ CHCl₃], and more preferably the palladium catalyst is palladium acetate, and
- a phosphine ligand, preferably selected from 1,1'-bis(diphenylphosphino)-ferrocene (DPPF), 1,2'-bis(diphenylphosphino)-ethane (DPPE), 1,3'-bis(diphenylphosphino)-propane (DPPP), 1,4'-bis(diphenylphosphino)-butane, and more preferably the phosphine ligand is 1,1'-bis(diphenylphosphino)-ferrocene.

2. The method of claim 1 wherein in step i)
- the dealkylating agent is selected from boron tribromide (BBr₃), aluminium iodide (AlI₃), aluminum bromide (AlBr₃), aluminum chloride (AlCl₃), pyridinium chloride (py⁺HCl), pyridinium bromide (py⁺HBr), lodotrimethylsilane (TMSI), and lithium diphenylphosphide (C₆H₅)PLi, and preferably the dealkylating agent is BBr₃, and/or
- ibogaine is reacted with the dealkylating agent in the presence of a solvent, preferably the solvent is selected from dichloromethane, chloroform, acetonitrile, toluene, and hexane, and more preferably the solvent is dichloromethane.

3. The method of claim 1 or 2 wherein in step i) the molar ratio of ibogaine to dealkylating agent is from 1.0:1.0 to 1.0:3.0, preferably is from 1.0:1.3 to 1.0:2.0 and more preferably from 1.5 to 1.8.

4. The method of any one of claims 1 to 3 wherein in step ii) the triflating agent is selected from *N*-phenyl-bis(trifluoromethanesulfonimide), *N*-(2-pyridyl)triflimide, *N-*phenyltriflimide, imidazole triflate, *N*-(2-pyridyl) triflimide, and preferably the triflating agent is *N*-phenyl-bis(trifluoromethanesulfonimide).

5. The method of any one of claims 1 to 4 wherein in step iii) the molar ratio of the reducing agent to 12-TfO-ibogamine is from 0.001:1.0 to 0.12:1.0, preferably from 0.01:1.0 to 0.10:1.0, more preferably from 0.01:1.0 to 0.09:1.0, and yet more preferably from 0.05:1.0 to 0.08:1.0.

6. The method of any one of claims 1 to 5 wherein step ii) and/or step iii) comprise purifying 12-TfO-ibogamine of step ii) and/or ibogamine of step iii) by Dry Column Vacuum Chromatography (DCVC), to provide purified 12-TfO-ibogamine and/or purified ibogamine.

7. The method of any one of claims 1 to 6 comprising after step iii) a further step iv) of crystallizing ibogamine or purified ibogamine provided in step iii) to provide a crystallized ibogamine.

8. The method of any one of claims 1-7 wherein
- the ibogaine of step i) is ibogaine hydrochloride and/or
- the ibogamine or the purified ibogamine provided in step iii) and/or the crystallized ibogamine provided in step iv) is free base ibogamine.

9. The method of claim 7 or 8 wherein crystallizing of step iv) comprises
- dissolving the ibogamine or the purified ibogamine obtained in step iii) in a solvent to provide a solution of ibogamine in the solvent, the solvent preferably being an alcohol, more preferably an alcohol selected from methanol, ethanol, isopropyl alcohol, and yet more preferably the solvent being methanol;
and
- crystallizing ibogamine from the solution of ibogamine in the solvent, to provide the crystallized ibogamine.

10. The method of claim 9 wherein ibogamine is dissolved in the solvent at a temperature from 30 to 80 °C, and more preferably from 40 to 60 °C.

11. The method of claim 9 or 10 wherein ibogamine is crystallized from the solution of ibogamine in the solvent by cooling the solution, preferably cooling the solution to a temperature from -50 to 0 °C, preferably from -40 to -5 °C, and more preferably from -30 to -10 °C.

12. A method for purifying ibogamine and/or an ibogamine-type compound selected from ibogaine, nor-ibogaine or 12-TfO-ibogamine, preferably for purifying ibogamine and/or 12-TfO-ibogamine, by DCVC, comprising eluting ibogamine and/or the ibogamine-type compound in the presence of ammonia.

13. The method of claim 12 wherein eluting is performed with a solvent system comprising a solvent, the solvent preferably selected from ethyl acetate and n-heptane, and ammonia, preferably the solvent comprising from 0.005 to 1 % v/v of ammonia, preferably from 0.05 to 0.5 % v/v and more preferably 0.08 to 0.2 % v/v of ammonia.

14. A method for crystallizing free base ibogamine comprising
- dissolving free base ibogamine in a solvent to provide a solution of ibogamine in the solvent, preferably the solvent being an alcohol, and more preferably being an alcohol selected from methanol, ethanol, and isopropyl alcohol, and yet more preferably the solvent being methanol;
and
- crystallizing free base ibogamine from the solution of ibogamine in the solvent, to provide a crystallized free base ibogamine.

15. The method of claim 14 wherein
- free base ibogamine is dissolved in the solvent at a temperature from 30 to 80 °C, more preferably from 40 to 60 °C;
and/or
- free base ibogamine is crystallized from the solution of free base ibogamine in the solvent by cooling the solution, preferably cooling the solution to a temperature from -50 to 0 °C, preferably from -40 to -5 °C, and more in particular from -30 to -10 °C.
